# EUROPEAN PATENT SPECIFICATION

(11) **EP 2 126 551 B1**
(45) Date of publication and mention of the grant of the patent: **10.08.2016**
(21) Application number: 08730081.0
(22) Date of filing: 18.02.2008
(51) Int. Cl.: G01N 21/78, G01N 33/28

(54) **ANALYSIS OF FUNCTIONAL FLUIDS**
ANALYSE FUNKTIONELLER FLÜSSIGKEITEN
ANALYSE DE FLUIDES FONCTIONNELS

(30) Priority: 28.02.2007 US 679928
(43) Date of publication of application: 02.12.2009
(73) Proprietor: The Lubrizol Corporation, Wickliffe, Ohio 44092-2298 (US)
(72) Inventor: MANKA, John S., Wickliffe, Ohio 44092-2298 (US)
(74) Representative: D Young & Co LLP
(86) International application number: PCT/US2008/054209
(87) International publication number: WO 2008/106337

(56) References cited:
- US-A- 2 770 530
- US-A- 6 072 890
- US-A1- 2005 227 369

## Description

### FIELD OF THE INVENTION

The present invention relates to the analysis of the quality or identity of functional fluids. In particular, the invention relates to using an aerosol, mist or spray delivery system for the reagent solution containing the indicator. This system comprises a reagent containing the indicator that produces a color in the presence of reactants such as basic components.

### BACKGROUND OF THE INVENTION

Functional fluids are employed in a variety of automotive, off-highway vehicles, on-highway vehicles, equipment, machines, metal working and industrial applications. It is important to know the identity, quality and condition of such functional fluids to prevent the improper and ineffective utilization of the functional fluid. A quality functional fluid insures that the condition of the device/equipment containing the functional fluid is productive and properly functioning. It is, therefore, desirable to monitor the identity, and the physical and/or chemical conditions of functional fluids.

Methods exist for the analysis of functional fluids using various reagents in determining the presence and/or concentration of various constituents of the functional fluids. Specific reagents may be employed for determining the presence and concentration of components in functional fluids. These methods generally analyze for pH, coloring agents, and contaminants using reactive reagents on test strips. These methods generally require controlled conditions. Further, these methods may be subjective and inaccurate.

Other methods and apparatus for assessing the quality of a used fluid include placing a measured amount of fluid upon an absorbent material, heating the sample and awaiting dispersion of the sample. The amount of undispersed sample may then be measured and rated quantitatively. These methods and apparatus require significant controlled conditions, including measurement of the fluid sample volume, the use of a template to measure and rate the quantity of undispersed the sample. Additionally these methods can include heating of the sample, and awaiting dispersal of the sample. Another method to analyze oil is disclosed in U.S. Pat. No. 5,313,824; to Hergruth, et al. comprising the steps of obtaining a sample of the oil, placing the sample upon the medium, maintaining the medium in a desired position for an effective period of time for the spot to be visible, visually comparing the spotted test medium against comparative visual indicia depicting lubricating oil in various conditions, and selecting the comparative example which most closely resembles the test medium spotted with the test sample. No chemical reaction occurs between the medium and the fluid, and it is just a visual observation of what the oil looks like compared to a standard.

Markers have been used to identify fluids. Proton accepting chemical substances, that at a solution concentration of below about 50 milligrams per liter, impart little or no significant color to organic solvents, have been proposed as markers, or taggants, especially for petroleum-derived fuels. The marker is dissolved in a liquid to be identified, and then subsequently detected by performing a chemical test on the marked liquid. Markers are sometimes employed by government agencies to ensure that the appropriate tax has been paid on particular grades of fuel. Oil companies also mark their products to help assist in identifying diluted or altered products. These companies often go to great expense to make sure their branded petroleum products meet certain specifications, for example, volatility and octane number, as well as to provide their petroleum products with effective additive packages containing detergents and other components. Consumers rely upon product names and quality designations to assure that the product being purchased is the quality desired. Thus, it is important to be able to identify a marker in a petroleum product.

Traditionally, the presence of a marker substance is detected and optionally quantified by extracting the fuel with an immiscible aqueous or significantly aqueous solution of an acid substance, the precise nature of which can be varied according to the characteristics of the marker substance. The acid reacts with the basic compound to produce a readily visible, more or less intensely colored cation, that is dissolved in the aqueous acid phase. This method is disclosed in U.S. Pat. No. 5,145,573. Additionally, a method has been disclosed in WO 03/078551 A2 where the acidic substance has been applied to a test strip. The test strip is dipped into the oil and diazo-type marker reacts with the acidic substance in the test strip and changes color.

US2770530 relates to an acid base composition for the testing of lubricating oils and for the determination of the time at which concentration of acidic contaminants in used oil has risen to a value such that the oil should be replaced. It further relates to a method of applying such composition to a used lubricating oil and the characteristics of the oil

US2005/227369 relates to a method to analyze the condition of a functional fluid comprising: (1) obtaining a sample of the used fluid; (2) placing the sample of the fluid to a test medium; (3) reacting the fluid with an indicator in the test medium; (4) analyzing visually the results of the reactor resulting in the determination of the condition of the fluid. Further an apparatus for analyzing functional fluids is disclosed in the form of a test medium consisting of an absorbent or nonabsorbent material which has been treated with a chemical indicator, marker substance or a developer or detector reagent upon which a sample of the fluid to be tested is placed.

US6072890 relates to the separation of lumber pieces that are of different species, wherein an indicator liquid is sprayed on to a fresh cut end of each lumber piece to produce a characteristic reaction, e.g. based upon pH. After a suitable interval the coated ends of the lumber pieces are optically scanned, the scanned image being analyzed spectrographically to identify the species of the lumber piece.

The quantity of marker substance in the extract may also be measured, for instance, by visible light absorption spectrophotometry, the results of which are then compared with a reference standard to determine the original concentration of basic marker in the fluid. It may be necessary to make repeated, typically two or three, extractions of the fluid to recover the entire amount of marker originally present in order for complete quantification. Additionally, the extracted, separated phase is classifiable as a hazardous waste and presents problems of safe and lawful disposal, especially when examinations are made "in the field." Furthermore, the fluid with which was tested may be contaminated, making return to its original source undesirable and presenting additional waste disposal problems.

It would be desirable to have an accurate and easy analytical method to determine the identity and/or the conditions of a functional fluid. It would further be desirable to have an accurate analytical method to determine the identity and/or the fluid condition in the field.

The present invention will rapidly indicate the identity of and/or the condition of a functional fluid such as lubricating oils, engine oil, automatic and manual transmission fluids, continuously variable transmission fluids, infinitely variable transmission fluids, greases, gear oils, hydraulic fluids, metalworking fluids, antifreeze fluids, coating system fluids, cooling systems fluids, farm tractor fluids, transformer fluids, fuels such as diesel, gasoline, biofuels, emulsified fuels, and the like in the field. Many owners/operators of equipment that depend on these functional fluids currently depend on standard guidelines, such as hours or mileage, to determine the appropriate interval to change the functional fluid or the end of its useful life. Additionally, labs are relied on today to determine the specific identify of a fluid, where a tool that would allow identification in the field would speed warranty resolution. Additionally, various absorbent materials (wipes, shop towels, paper towels, and napkins) are normally used in checking functional fluids. The present invention does not require the use of absorbent materials with a diagnostic functionality as the functional fluid can be used on any surface type material as long as the surface does not chemically interfere with the indicator.

A need exists for a simple and rapid method of chemically analyzing a sample of a fluid on a qualitative basis to determine condition, origin or other useful property. The present invention will rapidly indicate the condition and/or identity of a functional fluid such as lubricating oils, engine oil, automatic and manual transmission fluids, continuously variable transmission fluids, infinitely variable transmission fluids, greases, gear oils, hydraulic fluids, metalworking fluids, antifreeze fluids, coating system fluids, cooling systems fluids, farm tractor fluids, transformer fluids, fuels such as diesel, gasoline, biofuels, emulsified fuels, and the like in the field.

Indicators have been used in analytical methods for monitoring fluids. Generally, redox indicators are sensitive in the presence of air more specifically oxygen and light. The instability of indicators has made them not useful for analytical tests that monitor fluid conditions. It would be desirable to have a stable delivery system resulting in a stable indicator that is easy to use to determine the condition of a functional fluid. A need exists for a simple and rapid method of chemically analyzing a functional fluid to determine its condition, quality, identity or other useful properties.

It is an object of this invention to provide an easy and convenient delivery system to accurately analyze the condition, quality and identity of a functional fluid. It is a further object of the invention to provide a method to analyze functional fluids rapidly in the field. It is still a further object of the invention to provide a delivery method such as an aerosol, mist, spray, liquid or semi liquid for a stable indicator that can identify reactant byproducts in a functional fluid which thus identifies the quality and condition of the functional fluid. It is still the object of the present invention to provide a method to test the quality or the identity of a functional fluid in the field rapidly by untrained personnel and without precision measurement. It is still a further object of the invention to provide a diagnostic kit for analysis of functional fluids rapidly in the field.

### SUMMARY OF THE INVENTION

The present invention provides a method to determine the condition and/or identity of a functional fluid comprising
(1) obtaining a sample of the functional fluid;
(2) placing the sample of the functional fluid on a medium;
(3) contacting the functional fluid sample with a reagent solution wherein the reagent solution comprises a redox indicator in combination with an acid reducing agent where the acid reducing agent comprises dithiophosphoric acid;
(4) allowing the indicator in the reagent solution to react with the functional fluid sample on a medium to produce a color change;
(5) analyzing the results of the reaction by determining or comparing the resultant color change; and
(6) determining the condition or identity of the functional fluid;
wherein the reagent solution is sprayed onto the sample using a pump type sprayer, an aerosol spray, or a mister.

### DETAILED DESCRIPTION OF THE INVENTION

The present invention provides a method for analyzing and monitoring the condition or identity of functional fluids. The functional fluids comes from innumerable sources, including internal combustion engines, stationary engines, turbines, transmissions, differentials, pumps, metalworking operations, cooling systems, and the like. The functional fluids include automatic transmission fluids, continuously variable transmission fluids, infinitely variable transmission fluids, traction drive transmission fluids, manual transmission fluids, power steering fluids, antifreeze fluids, lubricating oils, greases, crankcase lubricants, mineral oils, oils with Group 1, 2, 3 or 4 base oils, differential lubricants, turbine lubricants, gear lubricants, gear box lubricants, axle lubricants, farm tractor fluids, transformer fluids, compressor fluids, cooling system fluids, metal working fluids, hydraulic fluids, brake fluids, industrial fluids, fuels, continuously variable transmission fluid, infinitely variable transmission fluid, and the like. In one embodiment, the functional fluid is an automatic transmission fluid. In one embodiment, the functional fluid is a power steering fluid. In one embodiment, the functional fluid is an internal combustion fuel such as gasoline and/or diesel. In one embodiment, the functional fluid is compressor fluids such as air compressor lubricants and/or turbine lubricants. In one embodiment, the functional fluid is an internal combustion engine oil. In one embodiment the functional fluid is tested after some time in use.

In one embodiment, the analysis of an engine oil identifies the total base number (TBN) remaining in the engine oil. Total base number is a useful measure of the quality and life remaining in an engine oil. The depletion of total base number in the engine oil indicates that the useful life of the engine oil is ending and the engine oil needs to be replaced or additized.

The total base number of a typical passenger care motor oil is between about 4 and 9 TBN depending on the initial quality of the engine oil. The total base number of a diesel engine oil is typically between about 4 and 15 TBN depending on the initial quality of the engine oil. When the TBN of an internal combustion engine oil falls to levels about 2 TBN the oil is very close to the end of its useful life and should be replaced, or additized for proper utilization of the oils.

The method of the present invention employs an indicator that analyzes TBN of a functional fluid such as an engine oil. The TBN in the functional fluid that needs to be changed is at least less 0.5 TBN or equal to about 0 TBN unit, in another embodiment less than about 1.0 TBN, and in another embodiment less than about 2.0 TBN, and in another embodiment less than 4.0 TBN, calculated as milligrams of KOH per gram of sample.

In one embodiment, oxidation byproducts occur due to the oxidation of the functional fluid over its life. The analysis of the present invention detects the functional fluids' oxidized species or oxidation byproducts that result from use of the fluid itself. Generally, functional fluids contain oxidation inhibitors in additive packages to prevent and/or delay the oxidation of the functional fluid. The oxidation byproduct and/or oxidizing species generally build up in the functional fluid after the depletion of or reduction of oxidation inhibitors. The oxidizing species and/or oxidation byproducts in the functional fluid demonstrate that the condition of the functional fluid has exceeded its useful life and should be changed for proper utilization of the functional fluid.

The oxidation byproducts/oxidizing species that the redox indicator determines include hydroperoxides, peroxides, oxides of nitrogen, nitrogen oxides, and the like. In one embodiment, the oxidizing byproduct is a peroxide or a hydro peroxide. The method can determine one or more combinations of the oxidation byproducts and/or oxidizing species.

The concentration of oxidation byproducts/oxidizing species in the functional fluid is at least concentration greater than about 150 ppm, in another embodiment greater than about 30 ppm, in another embodiment greater than about 10 ppm, and in another embodiment greater than about 1 ppm calculated as hydrogen peroxide to determine the oxidation byproducts/oxidizing species.

### Reagents

The reagent solution comprises a redex indication in combination with an acid reducing agent where the acid reducing agent comprises dithiophosphoric acid.

The choice of reagent depends on the type of functional fluid being tested and/or the parameters being determined such as the concentration of acidic or basic components, presence or concentration of metals, oxidative/reductive potential, identity markers or the presence of specific components to name a few. Reagents for the purposes of this invention are substances, indicators and/or markers that enable the state of a chemical system to be characterized. The reagents function by a variety of mechanisms both in how the specific parameter is determined in how the indicator responds. The reagents function by a color change as seen through visual examination, colorimetry, photometry, fluorescence, chemiluminescence and the like. The indicators can be used alone or in combination. In one embodiment the indicator is stable.

The color of the indicator is chosen depending on the type of functional fluid being tested and/or the level of degradation of the functional fluid. Certain colors contrast strongly to the usual color of the functional fluid which is preferred. The choice of a suitable color may be determined by a particular application. For example, in one embodiment automatic transmission fluid for passenger cars is colored red for identification purposes. It would be inappropriate to use an indicator that turns red to indicate an unacceptable condition in the functional fluid of an automatic transmission fluid. For example, in an automatic transmission fluid, a selection of the color indication is in the range of varying blues to greens and mixtures thereof would be desired.

Redox indicators include neutral red, safranine T or O, indigo, indigo carmine, methylene blue, thionine, thymolindophenol, 2,6-dichlorophenolindophenol, gallocyanine, nile blue, variamine blue, diphenyl amine, diphenylamine-4-sulfonic acid, barium salt, tris(2,2dipyridyl)iron(II) sulfate, N-phenylanthranilic acid, ferroin, nitroferroin, 5,6-dimethylferroin, 4-amino-4'-methyldiphenylamine, diphenylbenzindine-disulfonic acid, o-dianisidine, 3,3'-dimethylnaphthidine, 3,3'-dimethylnaphthidine disulfonic acid, bis(5-bromo-1,10-phenanthroline) ruthenium(II)) dinitrate, tris(5-nitro-1,10-phenanthroline) iron(II) sulfate, Iron(II)-2,2',2'-tripyridine sulfate, tris(4,7-biphenyl-1,10-phenanthroline) iron(II) disulfate, o,m'-diphenylaminedicarboxylic acid setopaline, p-nitrodiphenylamine, tris(1,10-phenanthroline)-iron(II) sulfate, setoglaucine O, xylene cyanole FF, erioglaucine A, eriogreen, tris(2,2'-bipyridine)-iron(II) hydrochloride, 2-carboxydiphenylamine [N-phenyl-anthranillic acid], benzidine dihydrochloride, o-toluidine, bis(1,10-phenanthroline)-osmium(II) perchlorate, diphenylamine-4-sulfonate Na salt), 3,3'-dimethoxybenzidine dihydrochloride [o-dianisidine], ferrocyphen, 4'-ethoxy-2,4-diaminoazobenzene, N,N-diphenylbenzidine, diphenylamine, N,N-dimethyl-p-phenylenediamine, variamine blue B hydrochloride, N-phenyl-1,2,4-benzenetriamine, Bindschedler's green, 2,6-dichloroindophenol (Na salt), 2,6-dibromophenolindophenol, brilliant cresyl blue [3-amino-9-dimethyl-amino-10-methylphenoxyazine chloride], iron(II)-tetrapyridine chloride, Starch (soluble potato, I₃ present), gallocyanine (25°C), nile blue A [aminonaphthodiethylamino-phenoxazine sulfate], indigo-5,5',7,7'-tetrasulfonic acid (Na salt), Indigo-5,5',7-trisulfonic acid (Na salt), indigo-5,5'-disulfonic acid (Na salt), phenosatranine, Indigo-5-monosulfonic acid (Na salt), bis(dimethylglyoximato)-iron(II) chloride, induline scarlet, and the like. In one embodiment, the redox indicators are methylene blue, p-nitrodiphenyl-amine, N,N-diphenylbenzidine, diphenylamine, neutral red and the like. In one embodiment, the redox indicator is methylene blue.

The reagent solution may optionally include a catalytic metal ion of metals such as Cu, Fe, Mo or Mn. In some instances the amount of reducing agent is in excess of the redox indicator material, by a stoichiometric factor of 1.1, 1.5, 2 or 10 for example. The excess or reserve reduction potential adds additional stability to the indicator system.

In one embodiment, about 0.5 wt. % methylene blue is prepared in a solution of isopropyl alcohol. To that solution is added excess of about 2.5 equivalents of di (2-ethylhexyl) dithiophosphoric acid resulting in the methylene blue reduced to its colorless form (II). The excess of the dithiophosphoric acid forms a salt of the reduced methylene blue and stabilizes it. This solution is applied to a test medium. The solvent is evaporated to provide a redox indicator test medium used to effectively evaluate functional fluids for oxidizing species and/or oxidizing byproducts such as peroxide.

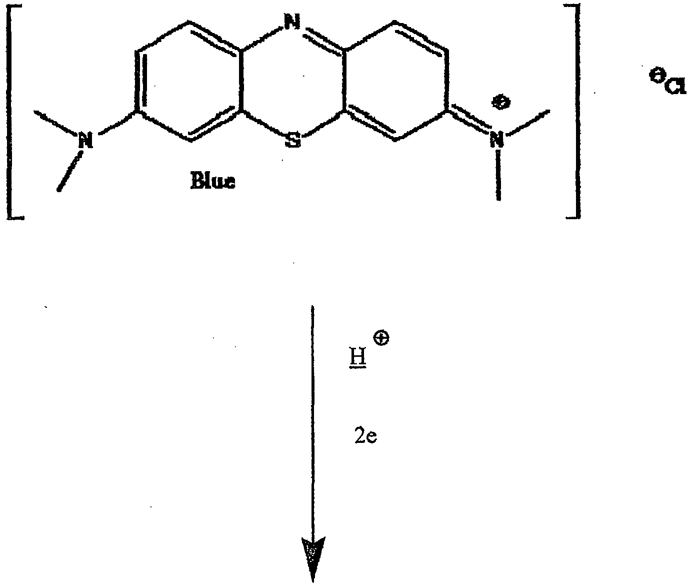

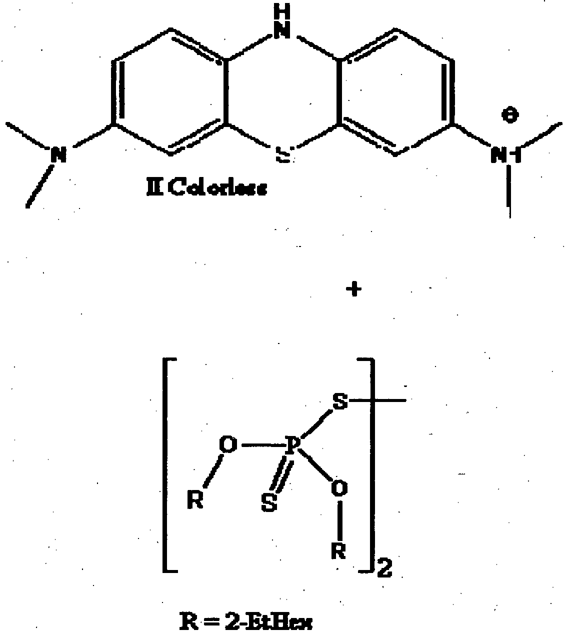

Marker substances include diazo dyes, anthraquinone dyes and the like, metals, metal salts, metal oxides, metal coordination complexes and the like or other substances compatible with the lubricant. It may be beneficial for the marker substance to be stable to the service conditions of the fluid, but it is not necessary. In general marker substances are used to identify new fluids. In some cases however, it could be useful to validate the identity of a functional fluid for, as an example, warranty claims. In this case the marker would need to survive and be detectable after experiencing the typical operating conditions of the functional fluid. Combinations of these substances may be used.

Developing agents are substances that will make conspicuous the presence or absence of a marker substance. Developing agents could include mineral or organic acids, organic or mineral bases or basic substances, oxidizing agents, reducing agents, chelating agents and the like. Combinations of developing agents may be used.

Optionally, in preparing the reagents, stabilizers may be added. The stabilizers include inhibitors such as para-amino benzoic acid, phenyl alphanapthal amines and the like. Another class of stabilizers includes acids such as hydrochloric acid, dithio-phosphoric acid, phosphoric acid, thiophosphoric acids. Another class of stabilizers includes bases such as sodium hydroxide, sodium bicarbonate, potassium hydroxide, and the like. Another class of stabilizers includes buffer solutions which are most commonly aqueous solutions of a weak acid and its conjugate base or a weak base and its conjugate acid, to maintain the pH of the reagents as necessary. The stabilizers can be used alone or in combination. The stabilizer is employed in the range 1 equivalent to or greater than the amount of indicator being reduced.

Each type of indicators can be used alone or in combinations. Further, the indicators may be a combination of indicators or one indicator. Each type of indicators are used in the range of about 0.001 wt. % to about 5 wt. %, and in another embodiment are used in the range of about 0.05 wt. % to about 2 wt. % and in another embodiment are used in the range of about 0.1 wt. % to about 1 wt % in the aerosol solution applied to the medium.

### Substrate or Medium

The functional fluid to be tested is placed upon any surface or medium. This surface or medium includes absorbent material, nonabsorbent material and combination thereof. The medium includes paper, cellulosic material such as cellulose, cellulose nitrate, cellulose acetate, cellulosic material, wood, paper, chromatography paper, filter paper, polymeric fibers, natural fibers, finely woven fabrics, metal, glass, glass micro fiber, sintered glass, silica and/or alumina coated surfaces such as thin layer chromatography plates, plastic, plastic laminated material, composites, cotton-(such as shop rags), cloth, and combinations thereof. Other absorptive/adsorptive materials, having the, general physical properties and characteristics of chromatography paper are also be acceptable. The medium must be capable of receiving a sample of the functional fluid but is not necessarily absorbent. The medium should be compatible with the specific indicator and/or developing agent; that is, in one embodiment it should not promote oxidation or acid-base reactions.

In one embodiment the preferred medium includes "Whatman" white colored chromatography paper or filter paper in the form of an easy to dispense and use wipe. In one embodiment, absorptive paper, such as chromatography paper is preferred, in particular for lubricating oil samples. Light colored chromatography paper provides a consistent background which contrasts well with the functional fluid, provides for a more conspicuous color change and has the proper adsorptive affinity for the various components of an oil. For example, the coloration of the indicator becomes more pronounced over time on the outer edges of the sample spot on the paper as the indicator colored portion of the mixture is swept along with the mobile phase (oil and solvent) faster than the darker components of the used oil, such as sludge. -This is due to the differences in adsorptive affinity for the paper. This difference in affinity becomes important as the concentration of sludge in the oil sample increases over the service life.

It is to be understood that depending on the type of functional fluid being analyzed and the particular functional purpose of the fluid, for instance, whether for gasoline powered engines as opposed to diesel powered engines, the test medium may need to be varied, whether the medium is chromatography paper or other type of paper, polymeric fiber material or nonabsorbent material like glass, plastic or metal. The medium may differ in its adsorptive affinity for the various components in the particular fluid, porosity, density, wicking ability, or other physical characteristics such as color.

Light colored media provides a consistent background which contrasts well with most functional fluids, and provides for a more conspicuous color change and has the proper adsorptive affinity for the various components of the functional fluid. For example, the coloration of the redox indicator becomes more pronounced over time on the outer edges of the sample spot on the paper as the indicator colored portion of the mixture is swept along with the mobile phase (functional fluid and solvent) faster than the darker components of the used functional fluid, such as sludge. This is due to the differences in adsorptive affinity for the paper. In one embodiment, the test medium is white. In one embodiment the medium includes "Whatman" white colored chromatography paper or filter paper in the form of an easy to dispense and use wipe.

The medium may differ in its adsorptive affinity for the various components in the particular functional fluid, such as porosity, density, wicking ability, or other physical characteristics such as color.

The shave of the medium is unimportant, so long as it is of an effective size to permit dispersion of the functional fluid sample, but small enough to be economical and limit waste.

### Solvents

Suitable solvents may be used with the reagents. The solvent used depends on the type of functional fluid being tested, the aerosol propellant and system (can, actuator and valve) being used and the indicator being used. Combinations of solvents are also useful when the indicator, depending on the application and type of analysis desired, is not soluble in the functional fluid. Particularly, solvents or combinations of solvents which present a desirable combination of properties including good solvency power and miscibility with the functional fluid and the redox indicator, low vapor pressure at ambient temperatures, high flash points and the like.

Solvents include aliphatic, unsaturated and aromatic hydrocarbons, alcohols, glycols, glycol ethers, lower alcohols, such as methanol, ethanol and propanol, ethers, esters, amides, water and the like. Combination of solvents may be used.

The solvent is used in the range of about 1% to about 99.9%, in one embodiment about 5% to about 98% and in another embodiment about 1% to about 95.5% of the reagent solution.

### Optional Components

Optional components may be added to the indicator solutions. These include surfactants to help media wetting, maskants and fragrances to improve customer appeal, antifoam additives to improve product manufacture and use, acids and bases to adjust the ph of the indicator solutions and buffers to maintain the pH of the indicator solutions. The optional components can be used alone or in combination.

Examples of surfactants include ionic, anionic (based on sulfate, sulfonate or carboxylate anions), sodium dodecyl sulfate (sds), ammonium lauryl sulfate, and other alkyl sulfate salts, sodium laureth sulfate, also known as sodium lauryl ether sulfate (sles), alkyl benzene sulfonate,-soaps, or fatty acid salts, cationic (based on quaternary ammonium cations), cetyl trimethylammonium bromide (ctab) a.k.a. hexadecyl trimethyl ammonium bromide, and other alkyltrimethylammonium salts, cetylpyridinium chloride (cpc), polyethoxylated tallow amine (poea), benzalkonium chloride (bac), benzethonium chloride (bzt), zwitterionic (amphoteric), dodecyl betaine, dodecyl dimethylamine oxide, cocamidopropyl betaine, coco ampho glycinate, nonionic, alkyl poly(ethylene oxide), alkyl polyglucosides, including: octyl glucoside, decyl maltoside, fatty alcohols, cetyl alcohol, oleyl alcohol, cocamide mea, cocamide dea, cocamide tea, neodol 25, fatty alcohols, ethoxylated alcohols, alkyl polyglucosides, triton bg-10 surfactant, triton cg-110 surfactant, branched secondary alcohol ethoxylates, tergitol tmn series, ethylene oxide / propylene oxide copolymers, tergitol I series, tergitol xd, xh, and xj surfactants, triton cf surfactants, triton df surfactants, tergitol minfoam surfactants, nonylphenol ethoxylates, tergitol np series, octylphenol ethoxylates, triton x series, secondary alcohol ethoxylates, tergitol 15-s series, triton ca surfactant, triton n-57 surfactant, triton x-207 surfactant, surfynol surfactants, primary amines, tertiary amines, monoalkyl and polyamines, ethoxylated amines, ethoxylated diamines, propoxylated amines, amine salts, quaternary ammonium salts, ethoxylated quaternary salts, propoxylated quaternary salts, amine oxides, amides, ethoxylated amides, esters: nonionic surfactants: ethoxylated fatty acids, amphoteric compounds, sulfosuccinates and sulfosuccinimates, fatty acid esters, fatty alcohols, alkanolamides, alkyl and alkyl ether sulfates, lauryl sulfates and lauryl ether sulfates, alkyl aryl sulfonates and alpha olefin sulfonates, alkoxylated nonionic surfactants, soya lecithins, alkyl sulfates, alkyl ether sulfates, imidazolines, alkanolamides, dowfax anionic surfactants, Dupont sulfonates, zonyl fluorosurfactants, peg esters and glyceryl esters, sorbitan esters/sorbitan ester ethoxylates, silicone surfactants, naphthalene condensates, sodium alkylnaphthalene sulfonates, pegol block copolymers, alkyl pyrrolidones, alkyl and glycol esters, emerest and trydet ethoxylated fatty acids and polyethylene glycol fatty acid esters, pilot hydrotropes , aristonate petroleum sulfonates, aristol sulfonatable oils, amido-amines, betaine amphoterics imidazolines imidazolinium amphoterics sulfosuccinates, fatty acid diethanolamides, neodol alcohols and the like. The surfactants can be used alone or in combination.

Examples of maskants and fragrances include abbarome 011, acalea tt, allyl amyl glycolate, ambrettolide, amyl cinnamic aldehyde, amyl salicylate, andrane, anethole 21/22, anethole usp, anethole usp, aphermate, apo patchone, bacdanol, benzyl butyrate, benzyl propionate, benzyl salicylate, bicyclononalactone, bornafix, canthoxal, cashmeran, cassiffix, cedramber, cedrenyl acetate, celestolide, cinnamalva, citral dimethyl acetal, intarome cotton odorsynthesis, intarome lavender musk odorsynthesis, citronalva, citronellol 700 jax, citronellol 750, citronellol 950, citronellol coeur, citronellyl acetate a, citronellyl acetate coeur, citronellyl acetate pure, citronellyl formate, clarycet, clonal, coniferan, cyclabute, cyclacet, cyclaprop, cyclemone a, cyclobutanate, cyclogalbaniff, cyclohexyl ethyl acetate, cyclohexyl ethyl alcohol, damascol 4, decyl methyl ether, delta damascone, dihydro cyclacet, dihydro floralate, dihydro floralol, dihydro myrcenyl acetate, dihydro terpineol, dihydro terpinyl acetate, dihydro terpinyl acetate dsa, dimethyl benzyl carbinol, dimethyl benzyl carbinyl acetate, dimethyl benzyl carbinyl butyrate, dimethyl cyclormol, dimethyl octanol, dimethyl phenyl ethyl carbinyl acetate, dimyrcetol, diola, dipentene 5100, dulcinyl recrystallized, ethyl ortho methoxy benzoate, ethyl phenyl glycidate, fleuramone, fleuranil, floralate, floralol, floralozone, fraistone, fructone, galaxolide 50 bb, galaxolide 50 dep, galaxolide 50 dpg, galaxolide 50 ipm, galbanum coeur, gelsone, geraldehyde, geraniol 5020, geraniol 7030, geraniol 980 pure, geraniol coeur, geranyl acetate a, geranyl acetate extra, geranyl acetate pure, grisalva, helional, herbac, hexalon, hexenyl salicylate, cis-3, hexyl acetate, hexyl cinnamic aldehyde, hexyl salicylate, hyacinth body, hyacinth body no.3, hydratropic-aldehyde dimethyl acetal, hydroxyol, hypo-lem, indolarome, indolene 50, intreleven aldehyde, intreleven aldehyde special, ionone 100%, ionone alpha, ionone alpha beta regular, ionone beta, iso amyl butyrate, iso amyl salicylate, iso bornyl propionate, iso butyl quinoline, iso cyclemone e, iso cyclo citral, iso cyclo geraniol, iso e super, isoproxen, jasmal, jasmelia, jessemal, kharismal, koavone, kohinool, lavonax, lemsyn, liffarome, lindenol, lyral, lyrame, lyrame super, maritima, meijiff, melafleur, methyl cedryl ketone chinese, methyl cinnamic aldehyde alpha, methyl ionone gamma a, methyl ionone gamma coeur, methyl ionone gamma pure, methyl lavender ketone, montaverdi, muguesia, muguet aldehyde, muguet aldehyde 50 bb50, myrac aldehyde, myrcenol super, myrcenyl acetate, neoproxen, nerol 800, nerol 850, nerol 900, neryl acetate jax, ocimene, ocimenyl acetate, octacetal, orange flower ether, orivone, orriniff 25% ipm, oxaspirane, ozofleur, pamplefleur, peomosa, phenafleur, phenoxanol, phenoxyethyl iso butyrate, phenoxyethyl propionate, phenyl ethyl acetate, phenyl ethyl alcohol, phenyl ethyl benzoate, phenyl ethyl formate, phenyl ethyl iso butyrate, phenyl ethyl salicylate, piconia, precyclemone b, prenyl acetate, proflora, pseudo linalyl acetate, reseda body, rosalva, rosamusk, roseate, rosemarel, salicynalva, sanjinol, santaliff, spirodecane, strawberiff, styralyl propionate, syvertal, terpineol 900, terpineol alpha jax, terpineol extra, terpinotene 20, terpinolene 90, terpinolene 90 pq, terpinyl acetate extra, terpinyl acetate jax, tetrahydro muguol, tetrahydro muguol coeur, tetrahydro myrcenol, tetrameran, tobacarol, triplal, unipine 60, unipine 85, vandor b, vanoris, verdol, verdox, verdox hc, verdural b extra, verdural extra, vertenex, vertenex hc, vertofix coeur, vertoliff, vigoflor, violiff, and mixtures thereof. also included in maskants are additives that act as odor scavengers such as amine scavengers and hydrogen sulfide scavengers like epoxides, basic amines, and the like. The maskants and fragrances can be used alone or in combination.

Examples of antifoams/defoamers are polysiloxanes, esters, insoluble oils, mineral oils, surfactants, amorphous silica, silicone emulsions, and the like. The antifoams/defoamers can be used alone or in combination.

Examples of acids include perchloric acid, hydroiodic acid, hydrobromic acid, hydrochloric acid, sulfuric acid, nitric acid, hydronium ion, chloric acid, bromic acid, perbromic acid, iodic acid, and periodic acid,fluoroantimonic acid, magic acid FSO₃HSBF₅, carborane superacid H(CHB₁₁CL₁₁), fluorosulfuric acid FSO₃H, triflic acid CF₃SO₃H , phosphoric acid, carboxylic acids, phenol, aromatic acids and the like. This can also include acidic buffer solutions. The acids can be used alone or in combination.

Examples of bases include potassium hydroxide, barium hydroxide, cesium hydroxide, sodium hydroxide, strontium hydroxide, calcium hydroxide, lithium hydroxide, rubidium hydroxide, butyl lithium, lithium diisopropylamide, sodium amide, sodium hydride, sodium carbonate, potassium carbonate, magnesium carbonate, ammonium carbonate, alanine, ammonia, NH₃ magnesium hydroxide, Mg(OH)₂, amine bases such as: methylamine, pyridine and the like. The bases can be used alone or in combination.

Examples of aqueous buffers include combinations of ammonium chloride and ammonia, formic acid and sodium format, acetic acid and sodium acetate, and the like. The aqueous buffers can be used alone or in combination.

The optional component is used in the range of about 0% to about 20%wt, in one embodiment about 0.01% to about 5%wt, and in another embodiment about 0.1 % to about 2%wt of the reagent solution.

### Propellants

The reagent solution can be sprayed onto the test media with a pump type sprayer or can be sprayed from an aerosol can. The propellant used must be compatible with the reagents. In one embodiment, the propellant is oxygen free or substantially free of oxygen. For example, with redox indicators the propellant should be oxygen free and chemically/oxidatively inert. Inert propellants include nitrogen, hydrocarbons, propane and butane, chlorofluorocarbons (CFCs), hydrocarbons, propane, n-butane and isobutene, dimethyl ether (DME) and methylethyl ether, nitrous oxide, hydrofluoroalkanes (HFA), HFA 134a (1,1,1,2-tetrafluorethane), HFA 227 (1,1,1,2,3,3,3-heptafluoropropane), saturated light hydrocarbons, C₃-C₆ (e.g., propane, isobutene, nbutane), CFC-11, HCFC-22, HCFC-142b, dimethyl ether CFC-11, HCFC-22, HCFC-142b, HCFC-152a, HFC-125, CFC-11, HCFC-22, HCFC-142b, HFC-227 ea, CFC-11, CFC-12, CFC-114, HCFC-22, HCFC-142b, compressed gases (carbon dioxide, air, nitrogen, nitrous oxide), SF6, fluorinated dimethyl ethers, Bis(difluoromethyl) ether, vinyl chloride monomer, and mixtures thereof. In one embodiment the propellant is free of halogens. Combinations of propellants may be used.

### Liquids/Semi Liquids

In a liquid or semi liquid delivery system, the reagent solution is in a liquid or semi liquid form. Generally, a liquid is a form of matter intermediate between gases and solids, in which the molecules are much more highly concentrated than gases, but much less than solids. Generally a semi liquid is a material that has an increased viscosity over a liquid substance. Generally, this increased viscosity is the result of the addition of materials that increase viscosity such as viscosity modifiers, polymers, tackifiers, clays, fillers, thickeners, rheology modifiers and the like. Semi liquids include gels, emulsions, suspensions, dispersions, additized liquids and the like. The container is any container capable of dispensing the liquid or semi liquid. The dispensing systems place the reagent solution onto the sample or in another embodiment the medium with the sample onto the reagent solution pumps without propellants, sprays without propellants, and the like.

### Visual Indicia

Analysis in particular qualitative analysis of the reacted test sample is accomplished by visual inspection of the reacted test sample using a provided visual indicia as a guide. Analysis occurs after an effective period of time to allow for the reaction between the components of the functional fluid and the reagents. Generally the time for reaction is in the range of about 0.01 sec. to about 1 hour, in another embodiment about 30 sec. minute to about 30 minutes, in another embodiment about 1 minute to about 15 minutes, and in another embodiment about 1 minute to about 5 minutes. In the embodiment, the time for the reaction is immediate.

The visual indicia include an artistic rendering, a reproduction of a photograph of a functional fluid in various conditions with and without the reagent; for example, oxidizing species and/or oxidization byproducts, color key, a written description of the-color change and the like. Combinations of visual indicia may be used. The visual indicia generally include one representation, two representations and more than two representations of the functional fluid disposed upon the media in various conditions with and without the reagent; for example, oxidizing species and/or oxidization byproducts. In one embodiment the preferred visual indicia is one in an unacceptable condition containing oxidizing species and/or oxidization byproducts and one in acceptable condition. A descriptive text corresponding to each of these examples may be provided.

In one embodiment the visual indicia depicted is dispersed upon the same or similar medium provided in the kit, to assure that the kit user compares the sample to be tested to examples produced under similar conditions. It is to be understood that a different number of indicia may be provided.

### Method

The method comprises the steps of (a)obtaining a sample of a functional fluid, (b) contacting the sample of the functional fluid with a medium, (c) spraying, misting, atomizing and/or contacting the appropriate reagent solution on the media that contains the sample of the functional fluid to be tested, (d) waiting for an effective period of time to allow for the reaction between the components of the functional fluid and the indicator in the reagent system, and (e) making a visual determination of the sample of the functional fluid on the medium using the printed instructions and/or comparative visual indicia depicting the functional fluid (in various) conditions

It is not necessary that the sample be taken during actual operation of the engine or other equipment or machinery in order to obtain a representative sample of the functional fluid. The sample of functional fluid may be taken at any time before, during or after operation of the engine or equipment. The functional fluid sample can be new, used or combinations thereof. In one embodiment the functional fluid test is especially useful during or after operation for some period of time.

The analysis of the condition of a functional fluid, for instance an automotive transmission fluid, is essentially dependent upon the reaction of components; i.e., oxidation byproducts in the functional fluid, with a redox indicator. For instance, automotive transmission fluid, as it is used, becomes oxidized forming oxidation byproducts and its inhibitor package becomes depleted, and there may be wear debris from the depletion of the anti-wear additives. During the service life of the automatic transmission fluid a point is reached where these components fall below a minimum acceptable level rendering the automatic transmission fluid unacceptable for further use. Continued use of an unacceptable automatic transmission fluid may cause damage to the transmission. When a sample of used automatic transmission fluid is obtained and the sample is placed upon a suitably treated medium, the oxidative inhibitor additives in the automatic transmission fluid, if they have been consumed or depleted allow for oxidative species in the fluid such as perioxide which will react with the redox indicator changing its color from colorless to various shades of blue or green depending on the concentration of the oxidizing species and presence of metal contaminants. The greater the concentration of oxidizing species, the more color change occurs. The presence or absence of a color change and the relative intensity of the color provides a means for qualitative analysis of the test sample.

For the analysis of the condition of a functional fluid, for instance as engine oil, under this invention is essentially dependent upon the reaction of components in the oil with an appropriate indicator. Engine oil, as it is used, becomes contaminated with acidic byproducts from oxidation, acidic components from fuel combustion and a component of sludge. The chemically basic additives, including detergents, which are added to the oil to neutralize these acidic components, are consumed over time. During the service life of the oil a point is reached where these basic components fall below a minimum acceptable level rendering the oil unacceptable for further use. Continued use of an unacceptable oil will likely cause damage to the engine. When a sample of used oil is obtained and the sample is placed upon a medium and treated with an indicator in a reagent solution, the basic additives in the oil, if they have not been consumed, will react with the indicator changing its color. The presence or absence of a color change and the relative intensity of the color provides a means for qualitative analysis of the test sample.

In one embodiment the functional fluid is an engine oil. The engine oil sample under ordinary circumstances may be obtained using a dipstick provided as a part of the engine, transmission or other equipment under lubrication. The user will withdraw an amount of oil along with the dipstick and the dipstick may then be wiped on the medium or the oil which will collect into a drop at the end of the dip stick may then placed upon the medium. Typically, less that 1 milliliter of oil is necessary for the analysis. Once the oil test sample has been placed on the medium, the sample spot is sprayed with the indicator aerosol and the indicator immediately begins to react. The user allows for an effective period of time to allow for the reaction between the components of the fluid and the indicator. Next, the user determines whether or not a color change occurred, and refers to the visual indicia as a guide. The user may consult the descriptive text accompanying the example selected to determine the condition or identity of the functional fluid.

The marking/identification of a functional fluid, for instance an automatic transmission fluid or automotive engine oil, is desirable because counterfeiting and adulteration/dilution of genuine functional fluids is a large concern of fluid suppliers as counterfeiting and adulteration results in a loss of profits, customer durability problems with vehicles, customer warranty claims, etc. due to a lack of adequate lubricant performance. A simple, easy to use marker system is beneficial since different functional fluids are usually indistinguishable visually. Chemical analyses or physical properties can tell various functional fluids apart but these analyses require expensive laboratory-test equipment and often take too long to be a practical end user identification test. The disclosed "lock" and "key" aerosol/spray technology enables an end user to exclude a counterfeit or adulterated product based on a color change resultant from the reaction between a known "lock" added to a functional fluid to yield a "marked" fluid and a "key" reagent present in the aerosol or spray which reacts with the "lock" reagent to give a predictable color change upon contact.

### EXAMPLES

A sample of the functional fluid to be tested is applied to a medium as described below. The results are analyzed visually after the functional fluid on the medium has reaction with the reagent in the aerosol, mist or liquids.

### Example 1 according to the invention

The Indicator solutions of a reagent is prepared at room temperature as follows:
(1) About a 0.05% wt. solution of methylene blue is prepared in isopropyl alcohol.
(2) To that solution is added excess di(2-ethylhexyl) dithiophosphoric acid (about 0.6% wt.).
(3) The methylene blue is reduced to its colorless form with corresponding formation of the disulfide of the dithiophosphoric acid.
(4) The excess dithiophosphoric acid forms a salt of (II) which stabilizes it.
(5) A sample of the functional fluid to be tested is placed on a blotter paper,
(6) The alcohol solution of the redox indicator is then applied to the sample containing media. The application of the indicator solution is sprayed on via a pump spray bottle.
(7) On exposure to hydroperoxides present in the functional fluid sample the aerosol indicator solution in contact with the sample containing media turns various shades of blue or green depending on the concentration of oxidizing species and the presence of metal contaminants (e.g., iron and copper ions).
(8) The user then analyzed the indicator visually and refers to the visual indicia as a guide.

### Example 2 not according to the invention

The indicator solutions of a reagent is prepared at room temperature as follows:
(1) About a 0.05% wt. solution of methylene blue is prepared in isopropyl alcohol.
(2) To that solution is added excess di(2-ethylhexyl) dithiophosphoric acid (about 0.6% wt.).
(3) The methylene blue is reduced to its colorless form with corresponding formation of the disulfide of the dithiophosphoric acid.
(4) The excess dithiophosphoric acid forms a salt of (II) which stabilizes it.
(5) A sample of the functional fluid to be tested is placed on Whatman chromatography paper.
(6) The alcohol solution of the redox indicator is then applied to the sample containing media. The application of the indicator solution is applied via a pipette.
(7) On exposure to hydroperoxides present in the functional fluid sample the indicator solution in contact with the sample containing media turns various shades of blue or green depending on the concentration of oxidizing species and the presence of metal contaminants (e.g., iron and copper ions).
(8) The user then analyzed the indicator visually and refers to the visual indicia as a guide.

### Example 3 according to the invention

Drain samples were taken from the automatic transmissions ("AT") of 29 vehicles and evaluated using the general method as in Example 1. A good correlation was observed between mileage on the AT fluids, wear metals (Fe, Pb, Cu) as determined by ICP emission spectroscopy analysis, and the observed colors produced on the media as in Example 1 as seen in Table 1.

**Table 1**

| | **Mileage on ATF** | **Total % Wear Metals** | **Reagent - Indicator Color** | **ASTM D664-04** |
|---|---|---|---|---|
| New ATF | 0 | -- | Red | -- |
| 2001 Ford Ranger | 2,000 | 0.0124 | Red | 2.03 |
| 2005 Honda Accord | 10,741 | 0.0069 | Red | 1.03 |
| 2001 Dodge Intrepid | 26,610 | 0.0095 | Red | 1.96 |
| 2003 Toyota Tacoma | 36,000 | 0.0187 | Red | 0.81 |
| 2003 Toyota Tacoma | 45,000 | 0.0150 | Red | 1.57 |
| 2001 Nissan Altima | 54,000 | 0.0183 | Red | 0.79 |
| 1996 Ford Winstar | 83,910 | 0.0350 | Blue | 1.01 |
| 2001 Ford winstar | 75,000 | 0.0398 | Green | 1.28 |
| 1996 Chrysler LHS | 74,131 | 0.0534 | Blue | 1.48 |
| 1997 Ford Taurus | 103,000 | 0.0757 | Green | 1.55 |
| 1999 Bodge Grand Caravan | 117,000 | 0.0510 | Green | 2.25 |

The total wear metals are the sum of the percents of Cu, Fe, and Pb in the automatic transmission fluid. A green result indicates a fluid in bad condition, blue result indicates a fluid that needs to be changed and red is a fluid in good condition.

### Example 4 not according to the invention

This example tests the quality of passenger car engine oil and consists of a substrate of "Whatman" filter paper and an aerosol indicator solution made up of ethanol or isopropanol (90% w/w), lauryl alcohol (10% w/w) and pH indicator (0.1% w/w). The pH indicator used is Alizarin (1,2-dihydroxyanthraquinone).

A used motor oil sample is placed on the dry "Whatman" filter paper and sprayed with the indicator solution. The basic additives (quantified as the TBN or Total Base Number, measured by ASTM D4739) of the oil react with the pH indicator inducing a color change from yellow to purple, with the indicator listed above, to a degree depending on the level of TBN. The intensity of the color change is reduced as the TBN drops over the service life of the oil until no purple color is apparent indicating the fluid has reached its maximum life. (See Table 2)

**Table 2-Effect of TBN on Color Change**

| **Age of Sample** | **Total Base Number (ASTM D4739)** | **Appearance of the Paper** | |
|---|---|---|---|
| | | **After 1 Minute** | **After 5 Minutes** |
| New | 5.6 mg KOH/g | Purple | Strong Purple |
| 2000 Miles | 4.1 | Faint purple | Purple |
| 6000 Miles | 1.9 | Brown spot, no purple apparent | Brown spot, no purple apparent |

The results in Table 2 demonstrate that an oil can be analyzed by visual indicia depicting the quality of the oil, as the brown color with no purple at 6000 miles indicates the condition of the oil.

### Example 5 not according to the invention

The marker and marker Indicator solutions were prepared at room temperature as follows:
Marker:
   (1) About 5000 ppmᵥₒₗ of reduced methylene blue marker reagent (per example 1) is added to a finished passenger car engine oil formulation.
Marker indicator Solution:
   (1) About a 3% wt. aqueous solution of hydrogen peroxide is placed in a pump type spray bottle.
   (2) The marked passenger car motor oil sample is placed on the dry "Whatman" filter paper and sprayed with the hydrogen peroxide indicator solution.
   (3) On exposure to the hydrogen peroxide solution, the media / marked engine oil turns blue to identify the engine oil as being marked. This marker color change positively identifies the engine oil.

### Example 6 not according to the invention

The Marker and marker Indicator solutions were prepared at room temperature as follows:
Marker:
   (1) About 5000 ppm of phenolphthalein is added to a finished passenger car engine oil formulation. The phenolphthalein marker was dissolved in a suitable solvent to help solubilize it in the functional fluid.
Marker Indicator Solution:
   (1) About a 0.5N solution of aqueous sodium hydroxide is placed in a pump type spray bottle.
   (2) The marked passenger car motor oil sample is placed on the dry blotter paper and sprayed with the basic indicator solution.
   (3) On exposure to the sodium hydroxide solution, the media / marked engine oil turns red to identify the engine oil as being marked. This marker color change positively identifies the engine oil.

## Claims

1. A method to determine the condition and/or identity of a functional fluid comprising
(1) obtaining a sample of the functional fluid;
(2) placing the sample of the functional fluid on a medium;
(3) contacting the functional fluid sample with a reagent solution wherein the reagent solution comprises a redox indicator in combination with an acid reducing agent where the acid reducing agent comprises dithiophosphoric acid;
(4) allowing the indicator in the reagent solution to react with the functional fluid sample on a medium to produce a color change;
(5) analyzing the results of the reaction by determining or comparing the resultant color change; and
(6) determining the condition or identity of the functional fluid;
wherein the reagent solution is sprayed onto the sample using a pump type sprayer, an aerosol spray, or a mister.

2. The method of claim 1 wherein the functional fluid is selected from the group consisting of automatic transmission fluids, engine oils, traction drive transmission fluids, manual transmission fluids, power steering fluids, antifreeze fluids, lubricating oils, greases, crankcase lubricants, mineral oils, oils with Group 1 ,2, 3 or 4 base oils, differential lubricants, turbine lubricants, gear lubricants, gear box lubricants, axle lubricants, brake fluids, farm tractor fluids, transformer fluids, compressor fluids, cooling system fluids, metal working fluids, hydraulic fluids, industrial fluids, fuels, continuously variable transmission fluid, infinitely variable transmission fluids, and mixtures thereof.

3. The method of claim 1 wherein the concentration of an oxidizing species in the functional fluid is at least a concentration greater than about 1 ppm calculated as hydrogen peroxide;
wherein the total base number (TBN) of the basic components in the functional fluid is at least greater than about 0.1TBN calculated as milligrams of KOH per gram of sample; and/or
wherein the reagent is used in the range of about 0.001 wt. % to about 5 wt % of the solution applied to the medium.

4. The method of claim 1 wherein the redox indicator is selected from the Group consisting of neutral red, safranine T or O, indigo, indigo carmine, methylene blue, thionine, thymolindophenol, 2,6-dichlorophenolindophenol, gallocyanine, nile blue, variamine blue, diphenyl amine, diphenylamine-4-sulfonic acid, barium salt, tris(2,2dipyridyl)iron(II) sulfate, N-phenylanthranilic acid, ferroin, nitroferroin, 5,6-dimethylferroin, 4-amino-4'-methyldiphenylamine, diphenylbenzindine-disulfonic acid, o-dianisidine, 3,3'-dimethylnaphthidine, 3,3'-dimethylnaphthidine disulfonic acid, bis(5-bromo-1,10-phenanthroline) ruthenium(II) dinitrate, tris(5-nitro-1,10-phenanthroline) iron(II) sulfate, Iron(II)-2,2',2"-tripyridine sulfate, tris(4,7-biphenyl-1,10-phenanthroline) iron(II) disulfate, o,m'-diphenylaminedicarboxylic acid setopaline, p-nitrodiphenylamine, tris(1,10-phenanthroline)-iron(II) sulfate, setoglaucine O, xylene cyanole FF, erioglaucine A, eriogreen, tris(2,2'-bipyridine)-iron(II) hydrochloride, 2-carboxydiphenylamine [N-phenyl-anthranillic acid], benzidine dihydrochloride, o-toluidine, bis(1,10-phenanthroline)-osmium(II) perchlorate, diphenylamine-4-sulfonate Na salt), 3,3'-dimethoxybenzidine dihydrochloride [o-dianisidine], ferrocyphen, 4'-ethoxy-2,4-diaminoazobenzene, N,N-diphenylbenzidine, diphenylamine, N,N-dimethyl-p-phenylenediamine, variamine blue B hydrochloride, N-phenyl-1,2,4-benzenetriamine, Bindschedler's green, 2,6-dichloroindophenol (Na salt), 2,6-dibromophenolindophenol, brilliant cresyl blue [3-amino-9-dimethyl-amino-10-methylphenoxyazine chloride], iron(II)-tetrapyridine chloride, starch (soluble potato, I₃ present), gallocyanine (25°C), nile blue A [aminonaphthodiethylamino-phenoxazine sulfate], indigo-5,5',7,7'-tetrasulfonic acid (Na salt), Indigo-5,5',7-trisulfonic acid (Na salt), indigo-5,5'-disulfonic acid (Na salt), phenosatranine, Indigo-5-monosulfonic acid (Na salt), bis(dimethylglyoximato)-iron(II) chloride, induline scarlet, and mixtures thereof.

5. The method of claim 1 wherein the medium is compatible with the reagent indicator and the medium comprises paper, cellulosic material, chromatography paper, filter paper, polymeric fibers, natural fibers, fabrics, polypropylene woven fabric, nonwoven fabric, metal, glass, plastic, composite materials, and combinations thereof.

6. The method of claim 1 wherein the reaction is in the range of about immediate to about 1 hour; and/or
wherein determining the condition of the functional fluid is selected from the group consisting of visually comparing the sample against a set of comparative visual indicia depicting the functional fluid and at least one different condition as a guide, using printed instructions as a guide, comparing photographs depicting at least one different condition, and combinations thereof.

7. The method of claim 1 wherein the reagent solution is sprayed by use of a propellant, wherein the propellant is selected from the group consisting of nitrogen, hydrocarbons, propane and butane, chlorofluorocarbons (CFCs), hydrocarbons, propane, n-butane, isobutene, dimethyl ether (DME), methylethyl ether, nitrous oxide, hydrofluoroalkanes (HFA), HFA 134a (1,1,1,2-tetrafluorethane), HFA 227 (1,1,1,2,3,3,3-heptafluoropropane), saturated light hydrocarbons, C₃-C₆, propane, isobutene, nbutane, CFC-11, HCFC-22, HCFC-142b, dimethyl ether CFC-11, HCFC-22, HCFC-142b, HCFC-152a, HFC-125, CFC-11, HCFC-22, HCFC-142b, HFC-227 ea, CFC-11, CFC-12, CFC-114, HCFC-22, HCFC-142b, compressed gases, carbon dioxide, air, nitrogen, nitrous oxide, SF6, fluorinated dimethyl ethers, Bis(difluoromethyl) ether, vinyl chloride monomer, and mixtures thereof.

8. The method of claim 1 comprising making a visual determination of the sample of the functional fluid on the medium using printed instructions and/or comparative visual indicia depicting the functional fluid in various conditions.

## Patentansprüche

1. Verfahren zum Bestimmen des Zustands und/oder der Identität eines funktionellen Fluids, umfassend
(1) Erhalten einer Probe des funktionellen Fluids;
(2) Platzieren der Probe des funktionellen Fluids auf einem Medium;
(3) Inkontaktbringen der Probe des funktionellen Fluids mit einer Reagenzlösung, wobei die Reagenzlösung einen Redoxindikator in Kombination mit einem Säure-Reduktionsmittel umfasst, wobei das Säure-Reduktionsmittel Dithiophosphorsäure umfasst;
(4) Erlauben, dass der Indikator in der Reagenzlösung mit der Probe des funktionellen Fluids auf einem Medium reagiert, um eine Farbänderung hervorzurufen;
(5) Analysieren der Ergebnisse der Reaktion durch Bestimmen oder Vergleichen der erhaltenen Farbänderung; und
(6) Bestimmen des Zustands oder der Identität des funktionellen Fluids;
wobei die Reagenzlösung unter Verwendung einer Pump-Typ-Sprühvorrichtung, eines Aerosolsprays oder eines Zerstäubers auf die Probe gesprüht wird.

2. Verfahren gemäß Anspruch 1, wobei das funktionelle Fluid ausgewählt ist aus der Gruppe bestehend aus Automatikgetriebefluiden, Motorölen, Reibradgetriebefluiden, Handschaltgetriebefluiden, Servolenkungsfluiden, Frostschutzfluiden, Schmierölen, Fetten, Kurbelgehäuseschmiermitteln, Mineralölen, Ölen mit Grundölen der Gruppe 1, 2, 3 oder 4, Differenzialschmiermitteln, Turbinenschmiermitteln, Getriebeschmiermitteln, Getriebekastenschmiermitteln, Achsenschmiermitteln, Bremsflüssigkeiten, Traktorfluiden, Transformatorfluiden, Kompressorfluiden, Kühlsystemfluiden, Metallbearbeitungsfluiden, Hydraulikfluiden, industriellen Fluiden, Kraftstoffen, Fluiden für stufenlos regelbare Getriebe, Fluiden für stufenlose Getriebe und Gemischen davon.

3. Verfahren gemäß Anspruch 1, wobei die Konzentration einer oxidierenden Spezies in dem funktionellen Fluid wenigstens eine Konzentration von größer als etwa 1 ppm, berechnet als Wasserstoffperoxid, ist;
wobei die Gesamtbasezahl (TBN) der basischen Komponenten in dem funktionellen Fluid wenigstens größer als etwa 0,1 TBN, berechnet als Milligramm KOH pro Gramm Probe, ist; und/oder
wobei das Reagens in dem Bereich von etwa 0,001 Gew.-% bis etwa 5 Gew.-% der auf das Medium aufgebrachten Lösung verwendet wird.

4. Verfahren gemäß Anspruch 1, wobei der Redoxindikator ausgewählt ist aus der Gruppe bestehend aus Nautralrot, Safranin T oder O, Indigo, Indigokarmin, Methylenblau, Thionin, Thymolindophenol, 2,6-Dichlorphenolindophenol, Gallocyanin, Nilblau, Variaminblau, Diphenylamin, Diphenylamin-4-sulfonsäure-Bariumsalz, Tris(2,2-dipyridyl)eisen(II)-sulfat, N-Phenylanthranilinsäure, Ferroin, Nitroferroin, 5,6-Dimethylferroin, 4-Amino-4'-methyldiphenylamin, Diphenylbenzindindisulfonsäure, o-Dianisidin, 3,3'-Dimethylnaphthidin, 3,3'-Dimethylnaphthidindisulfonsäure, Bis(5-brom-1,10-phenanthrolin)ruthenium(II)-dinitrat, Tris(5-nitro-1,10-phenanthrolin)eisen(II)-sulfat, Eisen(II)-2,2',2"-tripyridinsulfat, Tris(4,7-biphenyl-1,10-phenanthrolin)eisen(II)-disulfat, o,m'-Diphenylamindicarbonsäuresetopalin, p-Nitrodiphenylamin, Tris(1,10-phenanthrolin)eisen(II)-sulfat, Setoglaucin O, Xylencyanol FF, Erioglaucin A, Eriogrün, Tris(2,2'-bipyridin)eisen(II)-hydrochlorid, 2-Carboxydiphenylamin [N-Phenylanthranilinsäure], Benzidindihydrochlorid, o-Toluidin, Bis(1,10-phenanthrolin)osmium(II)-perchlorat, Diphenylamin-4-sulfonat-Na-Salz), 3,3'-Dimethoxybenzidindihydrochlorid [o-Dianisidin], Ferrocyphen, 4'-Ethoxy-2,4-diaminoazobenzol, N,N-Diphenylbenzidin, Diphenylamin, N,N-Dimethyl-p-phenylendiamin, Variaminblau-B-hydrochlorid, N-Phenyl-1,2,4-benzoltriamin, Bindschedler-Grün, 2,6-Dichlorindophenol (Na-Salz), 2,6-Dibromphenolindophenol, Brilliantcresylblau [3-Amino-9-dimethylamino-10-methylphenoxyazinchlorid], Eisen(II)-tetrapyridinchlorid, Stärke (löslich aus Kartoffel mit vorhandenem I₃), Gallocyanin (25 °C), Nilblau A [Aminonaphthodiethylaminophenoxazinsulfat], Indigo-5,5',7,7'-tetrasulfonsäure (Na-Salz), Indigo-5,5',7-trisulfonsäure (Na-Salz), Indigo-5,5'-disulfonsäure (Na-Salz), Phenosatranin, Indigo-5-monosulfonsäure (Na-Salz), Bis(dimethylglyoximato)eisen(II)-chlorid, Indulinscharlach und Gemischen davon.

5. Verfahren gemäß Anspruch 1, wobei das Medium mit dem Reagenzindikator vereinbar ist und das Medium Papier, Cellulosematerial, Chromatographiepapier, Filterpapier, Polymerfasern, natürliche Fasern, Gewebe, Polypropylengewebe, Vliesmaterial, Metall, Glas, Kunststoff, Verbundmaterialien und Kombinationen davon umfasst.

6. Verfahren gemäß Anspruch 1, wobei die Reaktion in dem Bereich von etwa sofortig bis etwa 1 Stunde liegt; und/oder
wobei das Bestimmen des Zustands des funktionellen Fluids ausgewählt ist aus der Gruppe bestehend aus visuellem Vergleichen der Probe mit einem Satz von visuellen Vergleichsindizien, die das funktionelle Fluid und wenigstens einen anderen Zustand als Orientierungshilfe zeigen, Verwenden von gedruckten Anleitungen als Orientierungshilfe, Vergleichen von photographischen Aufnahmen, die wenigstens einen anderen Zustand darstellen, und Kombinationen davon.

7. Verfahren gemäß Anspruch 1, wobei die Reagenzlösung unter Verwendung eines Treibmittels versprüht wird, wobei das Treibmittel ausgewählt ist aus der Gruppe bestehend aus Stickstoff, Kohlenwasserstoffen, Propan und Butan, Chlorfluorkohlenstoffen (CFCs), Kohlenwasserstoffen, Propan, n-Butan, Isobuten, Dimethylether (DME), Methylethylether, Stickstoffoxid, Hydrofluoralkanen (HFA), HFA 134a (1,1,1,2-Tetrafluorethan), HFA 227 (1,1,1,2,3,3,3-Heptafluorpropan), gesättigten leichten Kohlenwasserstoffen, C₃-C₆, Propan, Isobuten, n-Butan, CFC-11, HCFC-22, HCFC-142b, Dimethylether CFC-11, HCFC-22, HCFC-142b, HCFC-152a, HFC-125, CFC-11, HCFC-22, HCFC-142b, HFC-227 ea, CFC-11, CFC-12, CFC-114, HCFC-22, HCFC-142b, komprimierten Gasen, Kohlendioxid, Luft, Stickstoff, Stickstoffoxid, SF6, fluorierten Dimethylethern, Bis(difluormethyl)ether, Vinylchlorid-Monomer und Gemischen davon.

8. Verfahren gemäß Anspruch 1, umfassend das Durchführen einer visuellen Bestimmung der Probe des funktionellen Fluids auf dem Medium unter Verwendung von gedruckten Anleitungen und/oder visuellen Vergleichsindizien, die das funktionelle Fluid in verschiedenen Zuständen darstellen.

## Revendications

1. Procédé de détermination de l'état et/ou l'identité d'un fluide fonctionnel comprenant les étapes consistant à
(1) obtenir un échantillon du fluide fonctionnel ;
(2) placer l'échantillon du fluide fonctionnel sur un milieu ;
(3) mettre en contact l'échantillon de fluide fonctionnel avec une solution de réactif, la solution de réactif comprenant un indicateur redox en combinaison avec un agent réducteur acide, l'agent réducteur acide comprenant l'acide dithiophosphorique ;
(4) laisser l'indicateur dans la solution de réactif réagir avec l'échantillon de fluide fonctionnel sur un milieu pour produire un changement de couleur ;
(5) analyser les résultats de la réaction par détermination ou comparaison du changement de couleur résultant ; et
(6) déterminer l'état où l'identité du fluide fonctionnel ;
dans lequel la solution de réactif est pulvérisée sur l'échantillon au moyen d'un pulvérisateur de type à pompe, un pulvérisateur d'aérosol ou un brumisateur.

2. Procédé de la revendication 1 dans lequel le fluide fonctionnel est choisi dans le groupe constitué de fluides de transmission automatique, huiles de moteur, fluides de transmission d'entraînement de traction, fluides de transmission manuelle, fluides de direction assistée, fluides antigels, huiles lubrifiantes, graisses, lubrifiants de vilebrequin, huiles minérales, huiles avec huiles de base du groupe 1, 2, 3 ou 4, lubrifiants de différentiel, lubrifiants de turbine, lubrifiants d'engrenage, lubrifiants de boîte de vitesse, lubrifiants d'essieu, fluides de frein, fluides de tracteur agricole, fluides de transformateur, fluides de compresseur, fluides de système de refroidissement, fluides de travail des métaux, fluides hydrauliques, fluides industriels, carburants, fluides de transmission à variation continue, fluides de transmission à variation infinie, et des mélanges de ceux-ci.

3. Procédé de la revendication 1 dans lequel la concentration d'une espèce oxydante dans le fluide fonctionnel est au moins une concentration supérieure à environ 1 ppm calculée en peroxyde d'hydrogène ;
dans lequel l'indice de base total (TBN) des composants basiques dans le fluide fonctionnel est au moins supérieur à environ 0,1 TBN calculé en milligrammes de KOH par gramme d'échantillon ; et/ou
dans lequel le réactif est utilisé dans la plage d'environ 0,001 % en poids à environ 5 % en poids de la solution appliquée au milieu.

4. Procédé de la revendication 1 dans lequel l'indicateur redox est choisi dans le groupe constitué des rouge neutre, safranine T ou O, indigo, carmin d'indigo, bleu de méthylène, thionine, thymolindophénol, 2,6-dichlorophénolindophénol, gallocyanine, Nil bleu, bleu de variamine, diphénylamine, acide diphénylamine-4-sulfonique, sel de baryum, sulfate de tris(2,2-dipyridyl)fer(II), acide N-phénylanthranilique, ferroïne, nitroferroïne, 5,6-diméthylferroïne, 4-amino-4'-méthyldiphénylamine, acide diphénylbenzindine-disulfonique, o-dianisidine, 3,3'-diméthylnaphtidine, acide 3,3'-diméthylnaphtidinedisulfonique, dinitrate de bis(5-bromo-1,10-phénanthroline)ruthénium(II), sulfate de tris(5-nitro-1,10-phénanthroline)fer(II), sulfate de fer(II)-2,2',2"-tripyridine, disulfate de tris(4,7-biphényl-1,10-phénanthroline)fer(II), acide o,m'-diphénylaminedicarboxylique-sétopaline, p-nitrodiphénylamine, sulfate de tris(1,10-phénanthroline)-fer(II), sétoglaucine O, xylènecyanole FF, érioglaucine A, eriogreen, chlorhydrate de tris(2,2'-bipyridine)-fer(II), 2-carboxydiphénylamine-[acide N-phényl-anthranillique], dichlorhydrate de benzidine, o-toluidine, perchlorate de bis(1,10-phénanthroline)-osmium(II), diphénylamine-4-sulfonate (sel de Na), dichlorhydrate de 3,3'-diméthoxybenzidine [o-dianisidine], ferrocyphène, 4'-éthoxy-2,4-diaminoazobenzène, N,N-diphénylbenzidine, diphénylamine, N,N-diméthyl-p-phénylènediamine, chlorhydrate de bleu de variamine B, N-phényl-1,2,4-benzènetriamine, vert de Bindschedler, 2,6-dichloroindophénol (sel de Na), 2,6-dibromophénolindophénol, bleu de crényle brillant [chlorure de 3-amino-9-diméthyl-amino-10-méthylphénoxyazine], chlorure de fer(II)-tétrapyridine, amidon (soluble de pomme de terre, I₃ présent), gallocyanine (25 °C), Nil bleu A [sulfate d'aminonaphtodiéthylamino-phénoxazine], acide indigo-5,5',7,7'-tétrasulfonique (sel de Na), acide indigo-5,5',7-trisulfonique (sel de Na), acide indigo-5,5'-disulfonique (sel de Na), phénosatranine, acide indigo-5-monosulfonique (sel de Na), chlorure de bis(diméthylglyoximato)-fer(II), écarlate d'induline, et des mélanges de ceux-ci.

5. Procédé de la revendication 1 dans lequel le milieu est compatible avec le réactif indicateur et le milieu comprend du papier, un matériau cellulosique, du papier de chromatographie, du papier filtre, des fibres polymères, des fibres naturelles, des tissus, un tissu tissé en polypropylène, un tissu non-tissé, un métal, du verre, un plastique, des matériaux composites, et des combinaisons de ceux-ci.

6. Procédé de la revendication 1 dans lequel la réaction est dans la plage d'approximativement immédiate à environ 1 heure ; et/ou
dans lequel la détermination de l'état du fluide fonctionnel est choisie dans le groupe constitué de la comparaison visuelle de l'échantillon à un ensemble de marquages visuels comparatifs représentant le fluide fonctionnel et au moins un état différent en tant que guide, l'utilisation d'instructions imprimées en tant que guide, la comparaison de photographies représentant au moins un état différent, et des combinaisons de celles-ci.

7. Procédé de la revendication 1 dans lequel la solution de réactif est pulvérisée au moyen d'un propulseur, le propulseur étant choisi dans le groupe constitué de l'azote, des hydrocarbures, le propane et le butane, des chlorofluorocarbones (CFC), des hydrocarbures, le propane, le n-butane, l'isobutène, l'éther diméthylique (DME), l'éther méthyléthylique, l'oxyde nitreux, des hydrofluoroalcanes (HFA), HFA 134a (1,1,1,2-tétrafluoréthane), HFA 227 (1,1,1,2,3,3,3-heptafluoropropane), des hydrocarbures légers saturés, C₃-C₆, le propane, l'isobutène, le n-butane, CFC-11, HCFC-22, HCFC-142b, l'éther diméthylique CFC-11, HCFC-22, HCFC-142b, HCFC-152a, HFC-125, CFC-11, HCFC-22, HCFC-142b, HFC-227ea, CFC-11, CFC-12, CFC- 114, HCFC-22, HCFC-142b, des gaz comprimés, le dioxyde de carbone, l'air, l'azote, l'oxyde nitreux, SF₆, des éthers diméthyliques fluorés, l'éther bis(difluorométhylique), le monomère de chlorure de vinyle, et des mélanges de ceux-ci.

8. Procédé de la revendication 1, comprenant la conduite d'une détermination visuelle de l'échantillon du fluide fonctionnel sur le milieu au moyen d'instructions imprimées et/ou de marquages visuels comparatifs représentant le fluide fonctionnel dans différents états.
